# EUROPÄISCHE PATENTSCHRIFT

(11) **EP 0 461 218 B1**
(45) Veröffentlichungstag und Bekanntmachung des Hinweises auf die Patenterteilung: **08.12.1993**
(21) Anmeldenummer: 91900357.4
(22) Anmeldetag: 20.12.1990
(51) Int. Cl.: A61K 31/505

(54) **ZUBEREITUNG EINES SALZES VON OXYPURINOL IN ORALER FORM ZUR BEHANDLUNG VON HYPERURICÄMIE**
OXYPURINOL SALT COMPOSITION FOR ORAL ADMINISTRATION FOR TREATING HYPERURECEMIA
COMPOSITION DE SEL D'OXYPURINOL A ADMINISTRATION PAR VOIE ORALE POUR LE TRAITEMENT D'HYPERURICEMIE

(30) Priorität: 28.12.1989 AT 2958/89
(43) Veröffentlichungstag der Anmeldung: 18.12.1991
(73) Patentinhaber: BIOKINET CHEMISCHES LABORATORIUM GESELLSCHAFT M.B.H., 1170 Wien (AT)
(72) Erfinder: NITSCHE, Veit, A-1190 Wien (AT)
(74) Vertreter: Haffner, Thomas M., Dr.
(86) Internationale Anmeldenummer: AT9000126
(87) Internationale Veröffentlichungsnummer: WO9109600

(56) Entgegenhaltungen:
- EP-A- 0 237 348
- US-A- 3 624 205
- Clinical Science, vol. 66, no. 3, 1984, M.I. Shaw et al., pp. 257-267

## Beschreibung

Die Erfindung bezieht sich auf eine oral applizierbare Zubereitung zur Behandlung von Hyperuricämie, sowie auf die Verwendung einer derartigen Zubereitung.

Für die Behandlung von Hyperuricämie und insbesondere in der Therapie der Gicht als Uricostatikum wurde in der Vergangenheit in erster Linie Allopurinol eingesetzt. Allopurinol wird mit einer vergleichsweise geringen Halbwertszeit von etwa 40 min metabolisiert, wobei als Hauptmetabolit Oxypurinol entsteht, welchem gleichfalls bereits harnsäuresenkende Wirkung zugeschrieben werden konnte. Untersuchungen von Oxypurinol haben nun ergeben, daß Oxypurinol in vitro eine schwächer hemmende Substanz für die Xanthinoxidase darstellt als Allopurinol, wobei in vivo die bekannte wesentlich schlechtere, klinische Wirksamkeit von Oxypurinol in erster Linie auf unvollständige Resorption von Oxypurinol aus dem Gastrointestinaltrakt zurückzuführen sein dürfte. Aus den genannten Gründen fand bisher nur Allopurinol in der Therapie der Gicht als Uricostatikum Verwendung.

Ein wesentlicher Nachteil der Verabreichung von Allopurinol besteht nun in erster Linie darin, daß die therapeutische Wirksamkeit durch die Halbwertszeit begrenzt ist und daß Metabolisierungsarbeit geleistet werden muß, um zu einem zwar gleichfalls wirksamen Metaboliten, nämlich Oxypurinol zu gelangen. Bei Oxypurinol handelt es sich darüberhinaus lediglich um einen Hauptmetaboliten und bei der Metabolisierung von Allopurinol werden weitere Metaboliten freigesetzt, über deren gegebenenfalls nachteilige Nebenwirkungen wenig bekannt ist. In jedem Fall muß jedoch Metabolisierungsarbeit vom Körper geleistet werden, wodurch es zu einer Teilblockade für andere lebenswichtige Entgiftungsprozesse kommen kann.

Die Erfindung zielt nun darauf ab, ein verträgliches, oral applizierbares Präparat, beispielsweise in Form von Kapseln, Tabletten, Dragees oder einer Lösung, zu schaffen, welches unmittelbar zu einer entsprechenden Bioverfügbarkeit führt, ohne daß hiefür vom Körper Metabolisierungsarbeit geleistet werden muß. Weiters zielt die Erfindung darauf ab, ein Präparat der eingangs genannten Art zu schaffen, welches sich durch bessere Langzeitwirkung und damit durch leichtere und präzisere Dosierbarkeit auszeichnet, wobei mit der verbesserten Langzeitwirksamkeit gleichzeitig darauf abgezielt wird, die Zahl der Darreichungen pro Tag ohne gefährliche Uberkonzentrationen bei der Einmalgabe zu verringern.

Zur Lösung dieser Aufgabe besteht die erfindungsgemäße, oral applizierbare, therapeutische Zubereitung zur Behandlung von Hyperuricämie im wesentlichen darin, daß sie als Wirkstoff ein Salz von Oxypurinol enthält. Dadurch, daß ein Salz des Oxypurinols eingesetzt wird, konnte erreicht werden, daß die Resorptionen und damit die Bioverfügbarkeit wesentlich verbessert wurde und insbesondere erreicht werden, daß eine nahezu vollständige Resorption aus dem Gastrointestinaltrakt beobachtet werden konnte. Als Salze kommen hiebei Salze mit anorganischen und organischen Kationen in Betracht, wie sie üblicherweise für die Herstellung von Arzneistoffen verwendet werden. Bei einem unmittelbaren Vergleich der als Wirkstoff-Salze von Oxypurinol enthaltenden Präparate mit gleichen Mengen Allopurinol bei gleicher Dosierung wurde gefunden, daß trotz gleicher Gesamtdosis ein über einen längeren Zeitraum konstanter biologisch wirksamer Blutspiegel erreicht werden konnte, wobei im Vergleich zu reinem Oxypurinol das Oxypurinolsalz bei gleich hoher Dosierung eine um 170% verbesserte relative Bioverfügbarkeit aufwies. Da bei unmittelbarer Verabreichung des Oxypurinolsalzes die Metabolisierungsarbeit, wie sie im Falle des Allopurinols zu leisten wäre, entfällt und zusätzliche Metaboliten nicht in die Blutbahn gelangen, kann auf der Basis eines Oxypurinolsalzes ein sicheres und exakt dosierbares Präparat zur Behandlung von Hyperuricämie geschaffen werden.

Mit Vorteil enthält die erfindungsgemäße, oral applizierbare, therapeutische Zubereitung ein Alkali-, Erdalkali- oder ein ggf. substituiertes Ammoniumsalz des Oxypurinols.

Besonders bevorzugt wird zur Bildung des Ammoniumsalzes des Oxypurinols als organisches Kation N-Methyl-D-glucamin eingesetzt. Bei Verwendung derartiger Salze mit organischen Kationen wird eine besonders leichte Löslichkeit des Oxipurinolsalzes erzielt, wobei gleichzeitig eine stark verbesserte, relative Bioverfügbarkeit in bezug auf reines Oxipurinol erzielt werden kann.

Die Erfindung bezieht sich weiters auf die Verwendung eines Alkali-, Erdalkali- oder Ammoniumsalzes des Oxypurinols zur Herstellung eines Pharmazeutikums zur Behandlung von Hyperuricämie. Bei der Verwendung zur Herstellung eines Pharmazeutikums wird das Salz des Oxypurinols mit üblichen Hilfsstoffen in eine oral verabreichbare Form gebracht, wobei zur Geschmacksabdeckung ein Lacküberzug eingesetzt werden kann. Die tägliche orale Dosis kann mit einem derartigen Präparat zwischen 200 und 400 mg gewählt werden, wobei beispielsweise nach Einnahme von 350 mg maximale Blutspiegel von 7,8 ± 1,5 µg/ml erreicht werden können, wodurch die pharmakologische wirksame Dosis mit vergleichsweise geringen Mengen des Oxypurinolsalzes erzielt werden kann. Für die Normalisierung pathologischer Harnsäurewerte hat es sich herausgestellt, daß von dem erfindungsgemäßen Oxypurinolsalz täglich lediglich etwa 300 mg eingenommen werden müssen.

Die Erfindung wird nachfolgend an Hand eines Ausführungsbeispieles zur Herstellung der Oxypurinolsalze näher erläutert.

### Beispiel 1:

1 M Oxypurinol wird in einer äquimolaren Menge 0,1N Natronlauge aufgelöst. Die Lösung wird einige Minuten bei Raumtemperatur gerührt und dann wird das Lösungsmittel im Vakuum bei einer Temperatur von 30 bis 35°C abdestilliert. Nachdem das gesamte Lösungsmittel abdestilliert wurde, bleibt das Produkt in Form von farblosen Kristallen als Reinprodukt zurück. Das Produkt wird in einem Vakuumexsikkator über Calziumsulfat aufbewahrt.
Elementaranalyse für C₅H₃N₄O₂Na:
- berechnet:: C 34,48% H 1,72% N 31,81%
- gefunden:: C 34,79% H 1,89% N 30,29%

NMR: zeigt eine charakteristische Bande für das C-Atom mit benachbartem Stickstoff und Kohlenstoff sowie negativ geladenem Sauerstoff.

### Beispiel 2:

Auf analoge Weise, wie in Beispiel 1 beschrieben, wurde auch das Dinatriumsalz des Oxypurinols hergestellt, indem eine 1M Lösung von Oxypurinol mit einer 0,2M Lösung von NaOH umgesetzt wurde. Auch in diesem Falle wurde das Dinatriumsalz des Oxypurinols in quantitativer Ausbeute erhalten.
Elementaranalyse für C₅H₂N₄O₂Na₂:
- berechnet:: C 30,61% H 1,02% N 28,28%
- gefunden:: C 31,41% H 1,07% N 26,8%

NMR: zeigt eine charakteristische Bande für das C-Atom mit benachbartem Stickstoff und Kohlenstoff sowie negativ geladenem Sauerstoff.

## Patentansprüche

1. Verwendung eines Alkali-, Erdalkali- oder gegebenenfalls substituierten Ammoniumsalzes des Oxypurinols zur Herstellung eines Pharmazeutikums zur oralen Behandlung von Hyperuricämie.

2. Verwendung nach Anspruch 1, dadurch gekennzeichnet, daß das Salz des Oxypurinols mit üblichen Hilfsstoffen in eine oral verabreichbare Form gebracht wird.

3. Verwendung nach Anspruch 1 oder 2, dadurch gekennzeichnet, daß zur Bildung eines Ammoniumsalzes des Oxypurinols als organisches Kation N-Methyl-D-glucamin eingesetzt wird.

## Claims

1. Use of an alkali, alkaline earth or optionally-substituted ammonium salt of oxypurinol for the preparation of a medicament for the oral treatment of hyperuricaemia.

2. Use according to claim 1, wherein the salt of the oxypurinol is formulated with conventional additives into an orally-administrable form.

3. Use according to claim 1 or 2, wherein N-methyl-D-glucamine is introduced as an organic cation for the preparation of an ammonium salt of the oxypurinol.

## Revendications

1. Utilisation d'un sel alcalin, alcalino-terreux ou éventuellement d'ammonium substitué de l'oxypurinol, dans la préparation d'un produit pharmaceutique pour le traitement par voie orale de l'hyperuricémie.

2. Utilisation suivant la revendication 1, caractérisée en ce que le sel d'oxypurinol peut être placé avec des adjuvants classiques dans une forme d'administration par voie orale.

3. Utilisation suivant la revendication 1 ou 2, caractérisée en ce que la N-méthyl-D-glucamine est utilisée comme cation organique pour la formation d'un sel d'ammonium de l'oxypurinol.
